# EUROPÄISCHE PATENTANMELDUNG

(11) **EP 3 536 235 A1**
(43) Veröffentlichungstag der Anmeldung: **11.09.2019**
(21) Anmeldenummer: 18160780.5
(22) Anmeldetag: 08.03.2018
(51) Int. Cl.: A61B 5/04, A61B 34/20, A61B 5/0488, A61B 34/00, A61N 1/08

(54) **VORRICHTUNGSSYSTEM ZUR INTRAOPERATIVEN ORTUNG VON NERVEN**

(71) Anmelder: inomed Medizintechnik GmbH, 79312 Emmendingen (DE)
(72) Erfinder: Wegner, Celine, 79106 Freiburg (DE); Krüger, Thilo, 79312 Emmendingen (DE); Schlett, Paul, 79110 Freiburg (DE); Mattmüller, Rudi, 79312 Emmendingen (DE)
(74) Vertreter: Börjes-Pestalozza, Henrich

(57) **Zusammenfassung**

Die Erfindung betrifft ein Vorrichtungssystem zur intraoperativen Ortung von Nerven oder Nervengeweben, welches zur automatisierten intraoperativen Messung der Reizbarkeit von Nerven oder Nervengeweben und zur Darstellung des Verlaufes eines oder mehrerer Nerven oder zur Lokalisierung (insbesondere funktionell relevanter) Nervengewebe eingerichtet ist und einen oder mehrere Sensoren zur Ableitung von ein oder mehreren elektrophysiologischen Signalen, die als Resultat einer Reizung von Nerven oder Nervengeweben stimulierter Organe auftreten, umfasst, sowie dessen intraoperative Verwendung zur Ortung von Nerven und Nervengeweben, und verwandte Erfindungsgegenstände; alles wie in der Anmeldung näher dargelegt.

## Beschreibung

Die Erfindung betrifft ein Vorrichtungssystem zur intraoperativen Ortung von Nerven oder Nervengeweben, welches zur automatisierten intraoperativen Messung der Reizbarkeit von Nerven oder Nervengeweben und zur Darstellung des Verlaufes eines oder mehrerer Nerven oder zur Lokalisierung (insbesondere funktionell relevanter) Nervengewebe eingerichtet ist und einen oder mehrere Sensoren zur Ableitung von ein oder mehreren elektrophysiologischen Signalen, die als Resultat einer Reizung von Nerven oder Nervengeweben stimulierter Organe auftreten, umfasst, sowie dessen intraoperative Verwendung zur Ortung von Nerven und Nervengeweben, und verwandte Erfindungsgegenstände.

Intraoperatives Monitoring (IOM) oder insbesondere intraoperatives Neuromonitoring (IONM) wird bei einer Reihe von chirurgischen Maßnahmen angewendet, um Informationen zur Erkennung oder insbesondere Vermeidung der Schädigung von Nerven durch den chirurgischen Eingriff während einer Operation zu erhalten. IONM ist inzwischen bei chirurgischen Eingriffen gut etabliert.

Das pelvine Neuromonitoring (pIOM) beispielsweise ist eine junge Methode des intraoperativen Monitorings, welche vor wenigen Jahren auf den Markt eingeführt wurde. Diese Technologie und Methode erlaubt die Identifikation, Überwachung und Schonung der Nerven des kleinen Beckens bei Eingriffen wie zum Beispiel der Totalen Mesorektalen Exzision zur Entfernung eines Rektumkarzinoms.

Die Methode basiert auf der ausschließlich elektrischen Stimulation der gefährdeten Nervenstrukturen mittels einer Handsonde und der elektromyografischen (EMG) Überwachung des internen analen Schließmuskels (IAS) unter gleichzeitiger Blasenmanometrie.

Die Grundzüge der Methode wurden bereits in einem deutschen Patent (DE102010019796 B4) und einer europäischen Patentanmeldung (EP2589410 A) beschrieben. Der Fokus dieser Anmeldung liegt vor allem auf der Kombination von IAS EMG und der Blasenmanometrie und der Filterung des Signals des IAS in einem Bereich unter 30 Hz, sowie der Methode / des Verfahrens und die Verwendung dieser Anordnung.

Andere bekannte Methoden zur intraoperativen Überwachung der Nervenintegrität betreffen beispielsweise Operationen am Hals (z.B. bei Schilddrüsenoperationen, Operationen an der Nebenschilddrüse) oder jegliche sonstige Operationen (z.B.in der Orthopädie, Neurochirurgie, Gefäßchirurgie, Allgemeinchirurgie, Hals-Nasen-Ohrenchirurgie, Gynäkologie, Urologie oder anderen Bereichen), die zur Schädigung von Nerven und Nervenbahnen führen können.

Bisherige Verfahren und Vorrichtungen basieren in aller Regel ausschließlich auf einer elektrischen Stimulation und (neben der Beobachtung beispielsweise von resultierenden Muskelbewegungen) der Ableitung resultierender elektrophysiologischer Erfolgssignale durch Sensoren, z.B. mittels Elektromyographie (EMG) oder evozierter Potentiale (EP).

Die gefundenen Informationen werden dann akustisch und/oder abstrakt auf Bildschirmen dargestellt.

Dies bedingt eine Reihe von Nachteilen: Zum einen müssen die verwendeten Elektroden sowohl für die Stimulation als auch für die Ableitung elektrophysiologischer Reize sorgfältig vorbereitet und platziert werden, zum anderen erlauben sie lediglich eine "Ja/Nein"-Entscheidung hinsichtlich der Schädigung von Nervenbahnen und nur eine lokal sehr begrenzte Identifikation von funktionell relevantem Gewebe. Eine genaue Lokalisierung ganzer Nervenbahnen oder zusammenhängender funktionell relevanter Gebiete ist mit ihnen nicht möglich.

Zum anderen bedingt die intraoperative Überwachung ein aufmerksames Beobachten des oder der verwendeten Bildschirme und lenkt somit den Blick des Operateurs stark vom eigentlichen Operationsbereich ab, und akustische Signale sind in der Regel nicht sehr detailreich.

Schließlich muss der Chirurg sich bisher den Nervenverlauf mittels "Trial and Error"-Prinzip schrittweise durch punktuelles Abtasten mit einer Handsonde erarbeiten. Eine Visualisierung des Nervenverlaufes oder funktionell relevanter Areale ist hierdurch nicht möglich. Der Anwender muss sich den Nervenverlauf selbst merken.

Es ist bekannt, Nervenbahnen mittels einer Handsonde für die optische Stimulation mittels LED zu reizen, siehe z.B. US2014/0052221. Auch ein Verfahren zur Laserstimulation der Nervi cavernosi bei der Prostatektomie ist bekannt, siehe z.B. US 2013/0166001. US 3,900,034 beschreibt die Photochemische Stimulation von Nerven mittels eines Photodiodenlasers. CN 203728848 (A) beschreibt ein Laserarray zur Stimulation von Nerven. Das LED- oder Laserlicht erzeugt eine Stimulierung oder Reizung im Körper, wenn das eingestrahlte Laserlicht auf einen Nerv trifft. EMG-Signale können zur Bestimmen der Reizung von Nerven verwendet werden.

(A) Vor diesem Hintergrund betrifft die vorliegende Erfindung ein Vorrichtungssystem zur intraoperativen Ortung von Nerven, welches zur automatisierten intraoperativen Messung der Reizbarkeit von Nerven oder Nervengewebe und zur Darstellung des Verlaufes eines oder mehrerer Nerven oder der Lage eines Nervengewebes eingerichtet ist und einen oder mehrere Sensoren zur Ableitung von ein oder mehreren elektrophysiologischen Signalen, die als Resultat einer Reizung (Stimulierung) von Nerven oder Nervengeweben stimulierter Organe auftreten, umfasst, wobei dieses System
- eine Einheit zur Steuerung einer Quelle von nervenreizauslösender elektromagnetischer Strahlung mit einer Wellenlänge im Bereich von 500 nm bis 3000 nm zur Reizung von Nerven und/oder einer Quelle elektrischer Impulse zur Nervenreizauslösung mittels elektrischer Impulse;
- eine Einheit zum Ableiten durch die Nervenreizauslösung hervorgerufener des oder der elektrophysiologischer Signale,
- eine Einheit zum Berechnen, Steuern und Auswerten und
- eine Einheit zum Darstellen, insbesondere Visualisieren und/oder zum akustischen Darstellen des oder der elektrophysiologischen Signale beinhaltet - vorzugsweise eingerichtet zur Visualisierung auf einem Bildschirm (Monitor) und/oder (parallel) im Operationsbereich -,
wobei zwei oder mehr der genannten Einheiten auch zu einer übergeordneten Einheit zusammengefasst sein können, und
dadurch gekennzeichnet ist, dass es
- (i) eine Quelle der elektromagnetischen Strahlung umfasst, die eine oder mehr linear oder zwei- oder dreidimensional verfahrbare Einzelquellen und/oder ein Array mehrerer Quellen elektromagnetischer Strahlung beinhaltet oder eine lokalisierbare Sonde als Quelle elektromagnetischer Strahlung beinhaltet; und vorzugsweise eine Ablenkeinheit für die elektromagnetische Strahlung der ein oder mehreren Quellen elektromagnetischer Strahlung vorliegt, um die elektromagnetische Strahlung gezielt in intraoperativ zu untersuchende Bereiche einzustrahlen (bevorzugt),
- und/oder (ii) (ferner) eine (vorzugsweise lokalisierbare, insbesondere automatisch lokalisierbare) Sonde als die Quelle elektrischer (Stimulations-) Impulse beinhaltet,
und dass es derart eingerichtet ist, dass die Visualisierung und/oder akustische Darstellung des oder der elektrophysiologischen Signale und so des Verlaufs mindestens eines Nerven und/oder des Ortes eines Nervengewebes im Operationsbereich (*in situ*) oder zusätzlich (bevorzugt) oder alleine unter Darstellung von zusätzlichen für die Operation relevanten weiteren Informationen erfolgt, und dass die Visualisierung mittels einer Vorrichtung für erweiterte oder gemischte Realität erfolgt.

Nachfolgend werden eine Reihe von Begrifflichkeiten näher bestimmt - die spezielleren Definitionen können dabei in allen Ausführungsformen der Erfindung anstelle von weiteren Begriffen verwendet werden, wobei einzelne, mehrere oder alle weitere Begriffe durch speziellere Begriffe ersetzt werden, was zu spezielleren Ausführungsformen der Erfindung führt, die hier ebenfalls als offenbart gelten.

Die Erfindung wie eingangs beschrieben und nachfolgend näher definiert ermöglicht vorteilhaft eine bessere und einfachere Erfassung des Verlaufes von Nerven im Operationsbereich (Situs) ohne unnötige Ablenkung und somit präzises chirurgisches Vorgehen, um eine Schädigung oder Beeinträchtigung von Nerven zu vermeiden, also ein einfaches intraoperatives Monitoring des oder der interessierenden Nerven.

Intraoperativ bedeutet, dass das Vorrichtungssystem vor allem zur Verwendung während einer Operation, darüber hinaus zusätzlich nach und/oder vor einem chirurgischen Eingriff eingerichtet ist.

Automatisiert bedeutet, dass das Vorrichtungssystem (durch geeignete Hard- und Software-Ausstattung) die Messung der Reizbarkeit von Nerven, vor allem jedoch die Darstellung der gewonnenen Messdaten, insbesondere die Visualisierung und/oder akustische Darstellung des oder der elektrophysiologischen Signale und so des Verlaufs mindestens eines Nerven oder des Ortes von Nervengeweben im Operationsbereich (*in situ*) oder zusätzlich (bevorzugt) oder alleine unter Darstellung von zusätzlichen für die Operation relevanten weiteren Informationen mindestens weitgehend ohne Beeinflussung dieser Darstellung durch einen Menschen während der Operation durchführt, so dass die Visualisierung mittels einer Vorrichtung für erweiterte oder gemischte Realität erfolgt.

Für die Operation relevante weitere Informationen sind beispielsweise, ob der Nerv vorhanden ist oder nicht, der zeitliche Verlauf von elektrophysiologischen Signalen, wie EMG, EEG, SEP oder MEP-Signalen, Kommentare zum OP-Verlauf und dessen Dokumentation, Warnsignale z.B. wenn ein Nerv oder ein Nervengewebe plötzlich abweichende Stimulierbarkeit (ermittelt über die elektrophysiologische Erfolgssignale, wie z.B. EMG, EEG, SEP oder MEP) zeigt, die auf eine erfolgte oder auf das Risiko einer bevorstehenden Schädigung der Nerven/Nervengewebefunktion hindeuten, Informationen zum Abstand von chirurgischen Werkzeugen zu einem Nerven oder Nervengewebe; oder ferner Informationen zum Narkosestatus, Informationen zu Kreislaufparametern eines Patienten; oder dergleichen.

Ortung bedeutet die Festlegung der Position (Lokalisierung), insbesondere eines Nerven oder Nervengewebes.

"Funktionell relevant" (für Gewebe oder Areale oder Gebiete) nimmt Bezug auf anatomische Strukturen, die für Körperfunktionen wie für die Motorik, Sensorik oder autonome Funktionen wichtig sind und deshalb während einer Operation geschützt werden müssen.

Anstelle von "Stimulierung" kann auch "Stimulation" stehen oder umgekehrt.

Die Begriffe "Nervenbahnen", "Nerv" oder "Nerven" beinhalten Nerven- oder Nervenbündel, der Begriff "Nervengewebe" beinhaltet auch Areale mit Nervenzellen, wie Ganglien oder Areale im Gehirn, die (bzw. deren Verlauf/Ort) mit der erfindungsgemäßen Vorrichtung dargestellt werden können.

Das Vorrichtungssystem kann beispielsweise insbesondere zur Reizung von Nerven (dieser Begriff impliziert hier auch Nervenbahnen) in der Nähe des Situs oder auch von Nervengewebe in dessen Nähe (beispielsweise zur Reizung im zentralen Nervensystem bei intrakranialen Operationen, z.B. im Bereich des Kortex oder von Nuclei, oder an peripheren Nerven, beispielsweise bei Operationen an der Wirbelsäule oder der Schilddrüse) und Ableitung der elektrophysiologischen Signale an von bestimmten Bereichen des zentralen oder peripheren Nervensystems innervierten Zielorganen eingerichtet sein, so dass beispielsweise eine Ortung und Visualisierung von bestimmten Bereichen des Kortex oder bestimmter Nerven, die für bestimmte Zielorgane (z.B. vor allem Muskeln, in erster Linie mittels EMG, ferner mittels Evozierter Potentiale wie SEP, oder ferner EEG-Signale) eine Reizung bewirken, möglich ist.

Durch das Auftreffen eines (elektrischen oder durch elektromagnetische Strahlung (insbesondere optisch) hervorgerufenen) Stimulus werden also Nerven oder (funktionell relevantes) Nervengewebe erregt, was zu einer korrespondierenden Antwort der innervierten Strukturen (vor allem Muskel) führt. Diese Antwort wird aufgenommen und durch eine zeitliche Korrelation dieser Information mit der Ortsinformation der Stimulationsquelle (Stimulationsort) (insbesondere unter Berücksichtigung von Verzögerungen aufgrund der Nervenleitgeschwindigkeit) kann ein Nervenverlauf rekonstruiert und in einer erweiterten oder gemischten Realität visualisiert oder funktionell relevantes Nervengewebe (z.B. im Kortex) markiert werden.

"Ferner" bedeutet, dass mit diesem Attribut versehene Merkmale weniger bevorzugt sind als solche ohne dieses Attribut.

Stimulierung und Reizung werden hier äquivalent verwendet.

Sensoren zur Ableitung von ein oder mehreren elektrophysiologischen Signalen, die als Resultat einer Reizung von Nerven oder Nervengeweben stimulierter (funktionell verbundener) Organe auftreten, umfassen insbesondere ein oder mehrere Elektroden zur Ableitung von EEG- oder vorzugsweise EP- oder insbesondere EMG-Signalen. EEG steht für Elektroenzephalographie, EMG für Elektromyographie. Ein oder mehrere Elektroden dienen als Masseelektroden.

Soweit von elektromagnetischer Strahlung die Rede ist, ist vorzugsweise solche mit Wellenlängen im UV-Bereich oder ab 380 nm bis jeweils 3000 nm, insbesondere solche mit einer Wellenlänge im Bereich von 500 nm bis 3000 nm gemeint.

Eine Einheit zur Steuerung einer Quelle von nervenreizauslösender elektromagnetischer Strahlung beinhaltet insbesondere eine Schaltung zur Einstellung und Kontrolle der Parameter der elektromagnetischen Strahlung, wie Pulsweite, Leistung, Frequenz, Anzahl von Pulsen oder Pulsform.

Diese Einheit kann auch direkt zur Erzeugung der elektromagnetischen Strahlung ausgestattet sein, wobei die Strahlung dann über lichtleitende Fasern oder Faserbündel an die eigentliche "Strahlungsquelle" oder direkt über eine Sonde an den Stimulierungsort geleitet werden kann.

Eine Einheit zum Ableiten physiologischer Signale beinhaltet (in der Regel austauschbare) Elemente (wie Stromkabel oder lichtleitende Kabel) zur Zuführung der elektrischen Ströme von Erfolgssignalen (beispielsweise elektrische Potentiale stimulierter Muskeln), Elemente zur Verarbeitung der Daten (Hard- und/oder Software) für die Ausgabe an die Einheit zum Darstellen und ggf. zum Speichern von solchen Daten (Informationen und Parameter) sowie in der Regel einen Verstärker.

Die Auswerteinheit ist vorzugsweise software- und/oder hardware-implementiert zur Sammlung und Auswertung der gewonnenen elektrophysiologischen Signale eingerichtet.

"Eingerichtet" bedeutet, dass ein entsprechender Erfindungsgegenstand durch Software- und/oder Hardware-Ausstattung als Mittel für die jeweils angegebene Funktion ausgestattet ist.

Zwei oder mehr der genannten Einheiten können auch zu einer übergeordneten Einheit (z.B. als IONM-System) zusammengefasst sein.

Als Quelle der elektromagnetischen Strahlung ("Strahlungsquelle") können eine oder mehrere linear oder zwei- oder dreidimensional verfahrbare Einzelquellen und/oder ein Array mehrerer Quellen elektromagnetischer Strahlung dienen oder eine lokalisierbare Sonde als Quelle elektromagnetischer Strahlung. Vorzugsweise liegt auch eine Ablenkeinheit für die elektromagnetische Strahlung der ein oder mehreren Quellen elektromagnetischer Strahlung vor, um die elektromagnetische Strahlung gezielt in intraoperativ zu untersuchende Bereiche einzustrahlen. Diese Ablenkeinheit kann in einer besonderen Ausführungsform der Erfindung automatisiert ausgeführt sein, sodass die elektromagnetische Strahlung automatisch in verschiedene Orte des zu untersuchenden Bereichs eingestrahlt und so eine automatische Abtastung des zu untersuchenden Bereichs ermöglicht wird. Das Muster der Orte, an denen die elektromagnetische Strahlung eingestrahlt wird, kann beispielsweise einem Gitter entsprechen, die Reihenfolge der Abtastung kann dabei zum Beispiel geordnet nacheinander oder in randomisierter Art erfolgen. Alternativ können auch weitere zum Beispiel sequenzielle Algorithmen für die Abtastung zum Einsatz kommen, die die Zeit, die für die Abtastung des zu untersuchenden Bereichs benötigt wird, verringern.
Ferner kann alternativ oder insbesondere zusätzlich eine lokalisierbare Sonde als Quelle elektrischer (Stimulations-) Impulse beinhaltet sein.

Wo "umfasst", "beinhaltet", "vorliegt" oder "enthält" bzw. deren Grundformen oder Pluralformen stehen, bedeutet dies, dass weitere Merkmale vorhanden sein können.

Durch Stimulation und Ortung von Nerven aufgrund von am Zielorgan erhaltenen elektrophysiologischen Signalen kann so die Integrität eines oder mehrerer Nerven und/oder die Lage des oder der Nerven oder die Lage und/oder die Integrität der nervalen Verbindung von Nervengeweben, z.B. zentralnervösen, wie Kortex-, Arealen oder Nuclei, zu Erfolgsorten dargestellt werden. Anders ausgedrückt: Beim Auftreffen des (elektrischen oder vorzugsweise durch elektromagnetische Strahlung ausgeübten) Stimulus auf Nerven oder Nervengewebe (beispielsweise im OP-Situs) werden diese erregt und eine resultierende Antwort aus den innervierten Muskeln abgeleitet. Dieses Signal wird mit der Ortsinformation der Stimulationsquelle korreliert, was die Visualisierung des kompletten Nervenverlaufes oder Nervengewebes ermöglicht.

Die Visualisierung von Nerven oder Nervengeweben im Operationsbereich (Situs) ermöglicht insbesondere auch, deren Integrität zu Beginn einer Operation, während einer Operation und/oder am Ende einer Operation zu überprüfen, was aufgrund der Einrichtung des Vorrichtungssystems ermöglicht ist.

Vorzugsweise ist das Vorrichtungssystem dafür vorgesehen, insbesondere eingerichtet, dass der Verlauf eines Nerven oder mehrerer Nerven oder die Lage (Ort) eines Nervengewebes während einer Operation dargestellt wird (was insbesondere geeignet ist, zu vermeiden, aufgrund der Kenntnis seines Verlaufes oder seines Ortes, den Nerv oder das Nervengewebe zu beschädigen).

Funktionell relevante Gebiete (wie Nerven oder Nervengewebe) können zum Beispiel in einer Brille für die erweiterte oder gemischte Realität (die vorzugsweise mit einem Laserschutz (z.B. Laserschutzglas) ausgerüstet sein kann) einblendbar sein oder eingeblendet werden und stellen sich somit für den Operateur überlappend mit dem Patienten/Operationssitus dar.

Auch die direkte Markierung auf dem Situs mit sichtbarem Licht ist möglich.

Bei der Stimulation mittels elektromagnetischer Strahlung, wie optischer Stimulation, als Spezialfall der Stimulation mittels elektromagnetischer Strahlung kann beispielsweise durch Verschiebung des Fokuspunktes von ein oder mehreren Lichtstrahlen auch tiefer gelegenes Nervengewebe stimuliert werden, was dann eine 3D-Darstellung ermöglicht. Alternativ kann auch durch die Variation der Laserenergie in verschiedenen Tiefen Nervengewebe stimuliert werden, was auch eine 3D-Darstellung ermöglicht.

Bei einer Sonde für elektrische Stimulation können über die Verlaufs- bzw. Ortsanzeige hinaus funktionell relevante Informationen auch direkt auf der Sonde z.B. mittels LED auf dem Schaft oder über sichtbares Licht auf dem Situs angezeigt werden. So können Parameter abgeleiteter (insbesondere EMG-) elektrophysiologischer Signale, wie Amplitude oder Latenz, dargestellt und damit über den Verlauf einer Operation beobachtet werden, und aus Veränderungen können Schlussfolgerungen auf die Intaktheit der Nervenstrukturen gezogen werden.

Bei erweiterter Realität können weitere relevante Informationen parallel zur Realität angezeigt werden, z.B. Informationen zur Intensität und/oder zum zeitlichen Intensitätsablauf (z.B. Latenz) abgeleiteter EMG-Signale.

Gemischte Realität bedeutet speziell eine Mischung von Elementen der Realität (z.B. OP-Situs oder dessen direkte Darstellung) und der virtuellen Realität (z.B. Darstellung eines mittels eines erfindungsgemäßen Vorrichtungssystems erhaltenen Nervenverlaufs/Gehirnareals) und kann auch erweiterte Realität und erweiterte Virtualität umfassen.

Lokalisierbare (navigierbare) Handsonden sind insbesondere solche, deren Ort und Orientierung mittels optischer, elektromagnetischer oder mechanischer Methoden bestimmt werden kann und die hierfür mit Elementen wie Ultraschallreflektoren oder IR-Strahlungsreflektoren oder Haltearmen oder weiteren optischen Lage- und Ausrichtungsfeststellung, beispielsweise optischen Markern, ausgestattet sind, und hierdurch eine Lageermittlung des Stimulationsortes einer Handsonde ermöglichen. Entsprechende Empfangseinheiten sind Ultraschallempfänger, Empfänger für elektromagnetische Strahlung wie IR-Empfänger oder Kameras. Vorzugsweise ermöglichen sie die automatische und kontinuierliche intraoperative Lokalisierung, dabei werden die Form, der Winkel, beispielsweise über Auswertung der Dickeninformation einer Sonde, die je nach Winkel entlang ihres Verlaufes zu- oder abnehmen kann, Abstände, Form und Lage bestimmter Markerpunkte oder Winkelinformationen der Gelenke des Haltearms berücksichtigt. Beispiele für geeignete Systeme sind chirurgische Navigationssysteme, wo zum Beispiel die Lage dreier kugelförmiger Marker, die mit einem chirurgischen Instrument unter festem Lagebezug verbunden sind, durch Reflektion von Ultraschall, elektromagnetische Strahlung wie IR-Strahlung oder mittels Kamera unter Auswertung der Geometrieverhältnisse eine kontinuierliche Verfolgung (Tracking) des Ortes (Position, wo sich das Instrument befindet) des Instruments ermöglichen. Solche Systeme sind beispielsweise von BrainLab, Scopis oder Stryker erhältlich. Die Ausführung des Instruments als Stimulationssonde zur Einstrahlung von elektromagnetischer Strahlung und/oder des Einbringens von elektrischer Reizung und simultaner Ableitung der durch die Reizung hervorgerufenen Reaktion des Zielorgans gemäß dieser Erfindung ermöglicht die Darstellung von Nervenverläufen oder funktionell relevanten Arealen.

Die vorstehenden Abschnitte betreffen in erster Linie Ausführungsformen einer erfindungsgemäßen Vorrichtung, die entsprechend eingerichtet sind. Doch können die entsprechenden intraoperativen Methoden gleichfalls mittels dieser Abschnitte definiert werden.

Vor diesem Hintergrund betrifft die Erfindung folgende weitere Ausführungsformen:
(B) In einer besonderen Ausführungsform betrifft die Erfindung ein Vorrichtungssystem wie vorstehend (insbesondere in Absatz (A)) dargelegt, dadurch gekennzeichnet, dass es zur Visualisierung unter Darstellung von zusätzlichen (weiteren) relevanten Informationen ausgewählt aus einer Amplitude oder zusätzlich Latenz des oder der elektrophysiologischen Signale, Parametern der Nervenreizauslösung (Stimulierung), der Tiefeninformation eines lokalisierten Nervs in Relation zur Oberfläche des Operationsbereiches, der Nähe einer Sonde in Relation zum Nerv, der Variation einer Amplitude oder zusätzlich Latenz des oder der elektrophysiologischen Signale über die Zeit, der Variation von Parametern der Nervenreizauslösung über die Zeit oder zwei oder mehr oder aller dieser Informationen eingerichtet ist, insbesondere unter Darstellung mittels eines Farbcodes, mittels numerischer Werte, mittels grafischer Elemente, mittels eingeblendeten Textes und/oder mittels Markierungszeichen neben oder auf dem Nervenverlauf oder auf dem Operationsbereich (als Spezialfall für weitere Information).
(C) Ein weiterer besonderer Erfindungsgegenstand betrifft ein Vorrichtungssystem wie vorstehend, insbesondere unter (A) und (B), definiert, dadurch gekennzeichnet, dass es eine Vorrichtung für erweiterte oder gemischte Realität, ausgewählt aus einer Brille mit Microdisplay (besonders bevorzugt), einem 3D-Projektor und einem 2D Projektor oder ferner eine Anbindung an einen Laparoskopie-Bildschirm, einen Bildschirm eines Operationsroboters oder ein Operations-Mikroskop enthält, und dass die Darstellung von für die Operation relevanten Informationen durch Einblendung der zusätzlichen Informationen auf ein (z.B. Video-) Bild des ermittelten Nervenverlaufes (insbesondere samt Situsdarstellung) oder direkt auf den Situs erfolgt, also das Vorrichtungssystem hierfür eingerichtet ist.
(D) Ein weiterer besonderer Erfindungsgegenstand betrifft ein Vorrichtungssystem wie vorstehend, insbesondere unter (A), (B) oder (C), definiert, dadurch gekennzeichnet, dass als Sensor(en) zur Ableitung des oder der elektrophysiologischen Signale ein oder mehrere solche für mindestens ein elektrophysiologisches Signal, insbesondere Elektromyogramm (EMG), umfasst sind und es somit für die Ableitung von ein oder mehreren elektrophysiologischen Signalen von glatter und/oder quergestreifter Muskulatur nach Reizung von peripheren oder zentralen Nerven eingerichtet ist.
(E) Ein weiterer besonderer Erfindungsgegenstand betrifft ein Vorrichtungssystem wie vorstehend, insbesondere unter (A), (B), (C) oder (D) definiert, dass als Sensor(en) zur Ableitung der physiologischen Signale ein oder mehrere solche für mindestens ein elektrophysiologisches Signal, insbesondere ein Evoziertes Potential (EP), umfasst sind und es somit für die Ableitung von ein oder mehreren elektrophysiologischen Signalen nach Reizung von peripherem oder zentralem Nervengewebe eingerichtet ist.
(F) Ein weiterer besonderer Erfindungsgegenstand betrifft ein Vorrichtungssystem wie vorstehend, insbesondere unter (A), (B), (C), (D) oder (E), definiert, dadurch gekennzeichnet, dass es als lokalisierbare Sonde zum Abgeben elektrischer (Stimulations-) Impulse eine lokalisierbare (navigierbare) Handsonde mit mindestens einer Elektrode und/oder eine die Bestimmung des Ortes einer elektrischen Stimulation ermöglichende Einrichtung (Anordnung), beinhaltet.
(G) Ein weiterer besonderer Erfindungsgegenstand betrifft ein Vorrichtungssystem wie vorstehend, insbesondere unter (A), (B), (C), (D), (E) oder (F) definiert, dadurch gekennzeichnet, dass es als lokalisierbare Sonde zum Abgeben von elektromagnetischer Strahlung eine lokalisierbare (navigierbare) Handsonde mit mindestens einer Quelle und/oder eine die Bestimmung des Ortes einer Stimulation durch elektromagnetische Strahlung ermöglichende Anordnung beinhaltet.
(H) Ein weiterer besonderer Erfindungsgegenstand betrifft ein Vorrichtungssystem wie vorstehend, insbesondere unter (A), (B), (C), (D), (E), (F) oder (G) definiert, dadurch gekennzeichnet, dass es als Quelle der elektromagnetischen Strahlung einen Laser umfasst.
(I) Ein weiterer besonderer Erfindungsgegenstand betrifft ein Vorrichtungssystem wie vorstehend, insbesondere unter (A), (B), (C), (D), (E), (F), (G) oder insbesondere (H) definiert, dadurch gekennzeichnet, dass es als Quelle elektromagnetischer Strahlung einen Laser in Form eines Feststofflasers, z.B. Nd/YAG-Laser, Titan:Saphir-Laser, Faserlaser und/oder insbesondere mindestens eine Laserdiode, umfasst.
(K) Ein weiterer besonderer Erfindungsgegenstand betrifft ein Vorrichtungssystem wie vorstehend, insbesondere unter (A), (B), (C), (D), (E), (F), (G) oder insbesondere (H) definiert, dadurch gekennzeichnet, dass es als Quelle elektromagnetischer Strahlung einen Laser in Form eines Gaslasers, z.B. Helium-Neon-Laser, Kohlendioxidlaser, Metalldampflaser oder Farbstofflaser, umfasst.
(L) Ein weiterer besonderer Erfindungsgegenstand betrifft ein Vorrichtungssystem wie vorstehend, insbesondere unter (A), (B), (C), (D), (E), (F), (G) oder insbesondere (H) definiert, dadurch gekennzeichnet, dass es als Quelle elektromagnetischer Strahlung einen Laser in Form eines Freie-Elektronen-Lasers beinhaltet.
(M) Ein weiterer besonderer Erfindungsgegenstand betrifft ein Vorrichtungssystem wie vorstehend, insbesondere unter (A), (B), (C), (D), (E), (F), (G), (H), (I), (J), (K) oder (L) definiert, dadurch gekennzeichnet, dass es eine Ablenkeinheit der elektromagnetischen Strahlung beinhaltet, die die Einstrahlung der elektromagnetischen Strahlung in verschiedene Bereiche des Situs ermöglicht und es sich hierbei um akustooptische Deflektoren, elektrooptische Deflektoren, Flash-LiDAR, einen Prismenscanner oder insbesondere einen Spiegelscanner mit Galvanometerantrieb handelt.
(N) Ein weiterer besonderer Erfindungsgegenstand betrifft ein Vorrichtungssystem wie vorstehend, insbesondere unter (M) definiert, dadurch gekennzeichnet, dass die Ablenkeinheit eingerichtet ist zur automatischen Abrasterung des interessierenden Bereichs durch die elektromagnetische Strahlung.
(O) Ein weiterer besonderer Erfindungsgegenstand betrifft ein Vorrichtungssystem wie vorstehend, insbesondere unter (M) oder (N) definiert, dadurch gekennzeichnet, dass der Nervenverlauf durch den interessierenden Bereich durch Korrelation der Ortsinformation der Ablenkeinheit mit dem mittels des oder den Sensoren abgeleiteten Signal berechnet wird.
(P) Ein weiterer besonderer Erfindungsgegenstand betrifft ein Vorrichtungssystem wie vorstehend, insbesondere unter (A), (B), (C), (D), (E), (F), (G) oder (H) definiert, dadurch gekennzeichnet, dass die Vorrichtung zur Visualisierung des Nervenverlaufs (direkt) im Operationsbereich (also auf dessen Oberfläche) eine Lichtquelle, insbesondere in Form eines Laser-Projektors oder eines Pilotlasers, umfasst.
(Q) Ein weiterer besonderer Erfindungsgegenstand betrifft ein Vorrichtungssystem wie vorstehend, insbesondere unter (A), (B), (C), (D), (E), (F), (G), (H), (I), (J), (K), (L), (M), (N) oder (P) definiert, dadurch gekennzeichnet, dass es ein oder mehrere Linsen zur Kollimation der elektromagnetischen Strahlen, insbesondere von Laserlicht, umfasst.
(R). Ein weiterer besonderer Erfindungsgegenstand betrifft ein Vorrichtungssystem wie vorstehend, insbesondere unter (Q) definiert, dadurch gekennzeichnet, dass es ein oder mehrere Linsen zur Formung eines Gauß-Stahls und eine Einrichtung zur Verschiebung der einen oder mehreren Linsen zur Verschiebung des Fokuspunktes der elektromagnetischen Strahlen, insbesondere von Laserlicht, in verschiedenen Tiefen des Operationsbereichs umfasst und so eine 3D-Darstellung der zu reizenden Nerven ermöglicht.
(S) Ein weiterer besonderer Erfindungsgegenstand betrifft ein Vorrichtungssystem wie vorstehend, insbesondere (Q) definiert, dadurch gekennzeichnet, dass es ein oder mehrere Linsen zur Formung eines Bessel-Gauß-Strahls der elektromagnetischen Strahlen, insbesondere von Laserlicht umfasst.
(T). Ein weiterer besonderer Erfindungsgegenstand betrifft ein Vorrichtungssystem wie vorstehend, insbesondere unter (Q) oder (S) definiert, dadurch gekennzeichnet, dass es eine Einrichtung zur Änderung der Leistung der elektromagnetischen Strahlung umfasst und so eine Ausbreitung des Stimulationseffektes in verschiedene Tiefen und eine 3D-Darstellung der zu reizenden Nerven ermöglicht.
(U) Ein weiterer besonderer Erfindungsgegenstand betrifft ein Vorrichtungssystem wie vorstehend, insbesondere unter (A), (B), (C), (D), (E), (F) oder (G) definiert, dadurch gekennzeichnet, dass die lokalisierbare Handsonde ein oder mehrere Anzeigeelemente aufweist, welche die Darstellung der relevanten weiteren Informationen ermöglichen, z.B. auf einem Bildschirm oder mittels ein oder mehrerer LEDs.
(V) Ein weiterer besonderer Erfindungsgegenstand betrifft ein Vorrichtungssystem wie vorstehend, insbesondere unter (A), (B), (C), (D), (E), (F), (G) oder (U) definiert, dadurch gekennzeichnet, dass es eine Stimulationssonde mit mindestens einer Lichtquelle, insbesondere in Form von ein oder mehreren LEDs oder einem Pilotlaser, umfasst zur Ausgabe von Signalen mittels sichtbarem Licht, basierend auf den abgeleiteten physiologischen Signalen, die als Resultat einer Reizung von Nerven auftreten.
(W). Ein weiterer besonderer Erfindungsgegenstand betrifft ein Vorrichtungssystem wie vorstehend, insbesondere unter (A), (B), (C), (D), (E), (F), (G), (H), (I), (K), (L), (M), (N), (O), (P), (Q), (R), (S), (T), (U) oder (V) definiert, dadurch gekennzeichnet, dass es eine Augmented oder Mixed Reality-Brille, vorzugsweise mit integriertem Laserschutz (z.B. Laserschutzglas), umfasst.
(X) Eine bevorzugte Ausführungsform der Erfindung betrifft ein wie oben beschriebenes Vorrichtungssystem, eingerichtet zur Stimulation mittels einer lokalisierbaren ("navigierbaren") Sonde (vorzugsweise Handsonde) (Stimulationssonde), die eingerichtet ist zur Abgabe von Stimulationsreizen mittels elektrischer Reizung und/oder Stimulation mittels elektromagnetischer Strahlung, insbesondere Laserlicht. Die Sonde kann vorzugsweise als Handsonde mit einer elektrischen Stimulationselektrode und/oder mit einem Leiter und einem Anschluss für Laserlicht und einem endständigen Laserlichtsaustrittsbereich nahe dem zu stimulierenden Ort im Situs ausgestattet sein und weist im Falle einer Handsonde vorzugsweise ein Handstück und ein oder mehrere Anschlüsse für elektrische und/oder laserlicht-basierte Reizzufuhr und optional einen Schaft auf. Die Geometrie kann optional eine Ortsbestimmung (Tracking) mittels Ultraschall, optisch (z.B. mittels IR oder einer Kamera) oder mechanisch (z.B. mittels eines verbundenen Arms) und für die Ortsbestimmung (Lage, Stellung und dergleichen) der Sonde aufweisen, die eine Korrelierung von Reizungsort und damit Nervenverlauf oder Nervengewebeposition und Anzeige derselben ermöglicht. Im Falle einer laserlichtbasierten oder insbesondere einer elektrischen Sonde kann diese auch mit einer Lichtanzeige (z.B. als LED, wie OLED, Minibildschirm, sonstige Lichtquelle) aufweisen, die im Falle einer erfolgreichen Stimulierung (erkennbar am Auftreten korrespondierender elektrophysiologischer Signale) aufleuchten und somit eine Erkennung des Verlaufes einer Nervenbahn oder der Position eines Nervengewebesbereiches durch Abfahren ermöglicht.
(Y) Ein weiterer bevorzugter Erfindungsgegenstand betrifft eine navigierbare (lokalisierbare) Stimulationssonde als solche, vorzugsweise in Form einer Handsonde wie unter X definiert, die zusätzlich einen Marker für die Navigation (beispielsweise in Form eines Elementes mit drei IR oder andere optische Strahlung reflektierenden Kugeln) beinhaltet.
(Z) In einer weiteren bevorzugten Ausführungsform der Erfindung kann (was auch als solches ein eigener Erfindungsgegenstand ist) eine AR-Brille auch einen Marker für die Navigation beinhalten, entweder zur Festlegung ihrer Position im Raum oder in Form einer stereoskopischen Kamera, die eine Lagepositionsbestimmung zum Operationsbereich erlaubt.
(AA) Ein weiterer besonderer Erfindungsgegenstand betrifft ein Vorrichtungssystem wie oben (insbesondere wie unter einem der Absätze (A) bis (W) beschrieben, dadurch gekennzeichnet, dass es mit einem Algorithmus zur Beschleunigung der Lokalisation von Nerven und/oder Nervengewebe ausgestattet ist.

Weitere Erfindungsgegenstände betreffen die intraoperative Verwendung eines Vorrichtungssystems oder einer Vorrichtung wie vor und nachstehend beschrieben zur Bestimmung des Verlaufs eines Nerven oder des Ortes eines Nervengewebes, vorzugsweise gleichzeitig von dessen Funktionalität/Integrität, optional unter Einblendung von weiteren Informationen wie oben beschrieben.

### Figurenbeschreibung

**Fig. 1** Schematische Darstellung eines exemplarischen erfindungsgemäßen Vorrichtungsystems
**Fig. 2** Schematische Darstellung eines exemplarischen erfindungsgemäßen Vorrichtungssystems mit IONM-System
**Fig. 3** Schematische Darstellung eines exemplarischen erfindungsgemäßen Vorrichtungssystems während einer Operation im Halsbereich zur Bestimmung des Verlaufs von Nerven
**Fig. 4** Schematische Darstellung eines exemplarischen erfindungsgemäßen Vorrichtungssystems während einer intrakranialen Operation zur Bestimmung des Ortes von Nervengeweben, wie Motor-Kortex-Arealen
**Fig. 5** Schematische Darstellung einer exemplarischen erfindungsgemäßen AR-Brille mit Anzeige des Verlaufs einer Nervenbahn und Anzeige von Zusatzinformationen
**Fig. 6** Schematische Darstellung eines exemplarischen optischen Aufbaus und einer exemplarischen Ablenkeinheit für elektromagnetische Strahlung, die in einem erfindungsgemäßen Vorrichtungssystem vorliegen kann
**Fig. 7** Schematische Darstellung einer exemplarischen erfindungsgemäßen Handsonde für Laserstimulation
**Fig. 8** Schematische Darstellung einer exemplarischen erfindungsgemäßen Stimulationssonde mit Erfolgsanzeige
**Fig. 9** Schematische Darstellung einer exemplarischen erfindungsgemäßen Stimulationssonde mit alternativer Erfolgsanzeige
**Fig. 10** Schematische Darstellung einer exemplarischen erfindungsgemäßen Stimulationssonde mit Licht zur Markierung
**Fig. 11** Schematische Darstellung einer exemplarischen erfindungsgemäßen Stimulationssonde mit Licht zur Markierung mit detaillierter Darstellung des Lichtaustrittsbereiches
**Fig. 12** Schematische Darstellung einer exemplarischen erfindungsgemäßen Stimulationssonde mit Laserstimulation und Trackingelement
**Fig. 13** Schematische Darstellung eines exemplarischen erfindungsgemäß verwendbaren Algorithmus für die automatisierte Abrasterung des Situs zur Darstellung von Nerven

Bevorzugte Ausführungsformen der Erfindung finden sich in der Beschreibung, auch in den Beispielen, und in den Ansprüchen, insbesondere den Unteransprüchen, die hier durch Bezugnahme inkorporiert werden.

Die nachfolgenden Beispiele dienen der Illustration der Erfindung, ohne sie einzuschränken, doch stellen sie zugleich besondere Ausführungsformen der Erfindung dar.

### Beispiel 1: Erfindungsgemäßes Vorrichtungssystem:

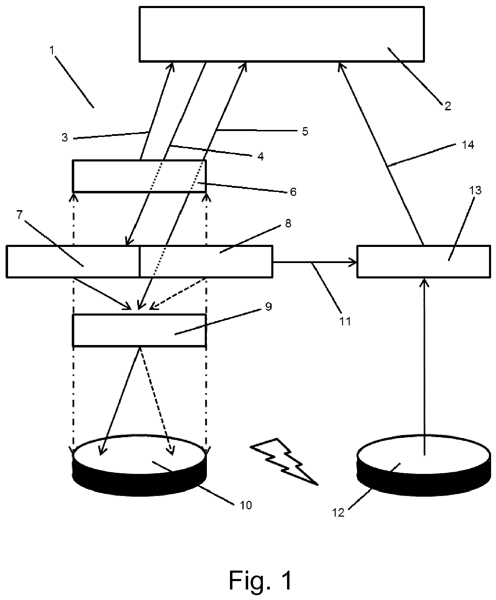

Fig. 1 zeigt ein exemplarisches Vorrichtungssystem 1 in stark vereinfachter Form, welches (als Beispiel für eine Einheit zur Steuerung, eine Einheit zum Auswerten und einer Einheit zum Darstellen wie vorstehend im allgemeinen Teil erwähnt) eine Kontrolleinheit 2 mit Einrichtung zur Darstellung von Nervenbahnen oder Nervengeweben umfasst. Eine Einheit zum Aufnehmen optischer Information, hier exemplifiziert in Form einer Kamera **6**, ermöglicht die Bilddatenübertragung **3** eines Bildes des Situs zur Kontrolleinheit. Weiterhin erfolgt eine Übertragung von Laserparametern (Laserparameterübertragung **4**) zur Kontrolle der Eigenschaften des Laserstrahls (z.B. Leistung, Pulsweite, Frequenz) und eine Übermittlung von Positionsdaten (Positionsdatenübertragung **5**) an einen Scanner **9** (z.B. Laserscanner, beispielsweise in Form eines Scankopfes) zur Steuerung des Einstrahlungsortes eines Lasers **7** als Stimulator. Die tatsächlich eingestellten Positionsdaten **5** werden von dem Scanner 9 (z.B. Laserscanner) an die Kontrolleinheit **2** zurückgegeben.

Bilddatenübertragung **3**, Laserparameterübertragung **4** und Positionsdatenübertagung **5** erfolgen über entsprechende Elemente des Vorrichtungssystems **1.**

Ein Laser **7** (die Laserquelle kann auch in die Kontrolleinheit **2** integriert sein) ermöglicht über einen Scanner (z.B. Scankopf) **9** eine Stimulation von Nerven oder Nervengewebe im funktionell relevanten Areal **10** (beispielsweise im Bereich eines Operationssitus). Ein optionaler Lasermarkierer **8** ermöglicht die optisch erkennbare Darstellung eines Nerven oder eines Nervengewebeareals direkt im funktionell relevanten Areal **10**. Unter Zeitinformationsübertragung **11** (die auch an anderer als der gezeigten Stelle erfolgen kann und mittels eines Zeitübertragungselementes erfolgt) werden Reaktionen, die am Zielorgan **12** (insbesondere von Muskeln oder Muskelgruppen) hervorgerufen werden, mit der Stimulation über den Laser **7** synchronisiert. Durch Kombination der Ortsinformationsübertragung **5** und der Zeitinformationsübertragung **11** kann die Darstellung eines stimulierten Nerven oder eines stimulierten Nervengewebes mittels des Lasermarkierers **8** (der auch ein Lichtmarkierer sein kann) direkt auf dem funktionell relevanten Areal **10** (insbesondere Situs einer Operation) oder mittels einer anderen Einheit zum Darstellen (z.B. einer Brille mit Microdisplay, ferner einem 3D-Projektor, einem 2D Projektor, einem Laparoskopie-Bildschirm, einem Bildschirm eines Operationsroboters oder einem Operations-Mikroskop) aufgrund der Erfolgsinformationsübertragung **14** (für die ebenfalls ein entsprechendes Element in dem Vorrichtungssystem **1** vorgesehen ist), deren Signalstärke durch einen Verstärker **13** vergrößert sein kann, erfolgen.

Die Darstellung kann dabei den Nervenverlauf oder den Ort des Nervengewebes im funktionell relevanten Areal **10** in farbig kodierter Form anzeigen - beispielsweise grün bei ungestörten EMG- oder ferner EEG-, SEP- oder EP-Signalen, gelb bei während der Operation sich zeigender Verschlechterung der Signale als Warnung, dass der Nerv oder das Nervengewebe durch den chirurgischen Eingriff gefährdet sind, oder rot bei völligem Abbruch der Reizbarkeit des Nerven wegen des chirurgischen Eingriffes - dann wird nur noch der gespeicherte Wert des Ortes angezeigt.

Neben (bevorzugt) oder anstelle der Verwendung des Scankopfes **9** können auch eine oder mehrere Handsonden für elektrische oder auf elektromagnetischer Strahlung beruhende Stimulatoren (siehe Beispiele unten) für die Stimulation der Nerven oder Nervengewebe vorgesehen sein.

### Beispiel 2: Beispiel für erfindungsgemäßes Vorrichtungssystem mit IONM-System

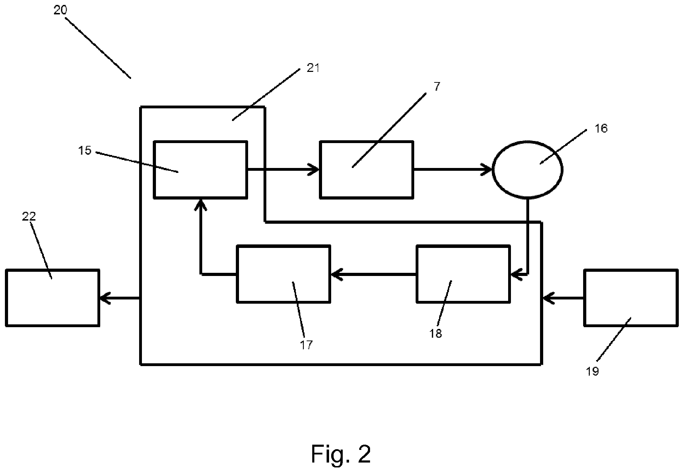

Fig. 2 zeigt als Schema Vorrichtungssystem **20** eine exemplarische Ausführungsform eines erfindungsgemäßen Vorrichtungssystems **1**, welches ein Interoperatives Neuromonitoring-System - IONM-System **21** beinhaltet. Rein exemplarisch erfolgt die Abgabe von elektromagnetischer Strahlung als Stimulus **15** mit einer Abtastfrequenz (beispielsweise von 100 Hz) über einen Laser **7** auf den Situs **16** unter optischer Erfassung **19** über eine EMG-Ableitung **18** unter Verwendung von einem Zähler **17** mit (beispielsweise 10 ms) Pause zwischen den einzelnen Stimuli **15**.

Die Nerven- oder Nervengewebe-Darstellung **22** erfolgt mittels einer entsprechenden Einheit in Form eines Lasermarkierers **8** (Fig. 1) (bevorzugt), einer Brille mit Microdisplay für einen Operateur (sehr bevorzugt), oder ferner einem 3D-Projektor, einem 2D Projektor, einem Laparoskopie-Bildschirm, einem Bildschirm eines Operationsroboters oder einem Operations-Mikroskop.

Das IONM-System **21** beinhaltet die EMG-Ableitung **18**, den Zähler **17** und den Geber für den Stimulus **15,** sowie eine Vorrichtung zur Verarbeitung und Korrelation der Daten.

### Beispiel 3: Erfindungsgemäßes Vorrichtungssystem zur Ermittlung des Verlaufes (Ortung) von Nerven bei einer Operation im Halsbereich

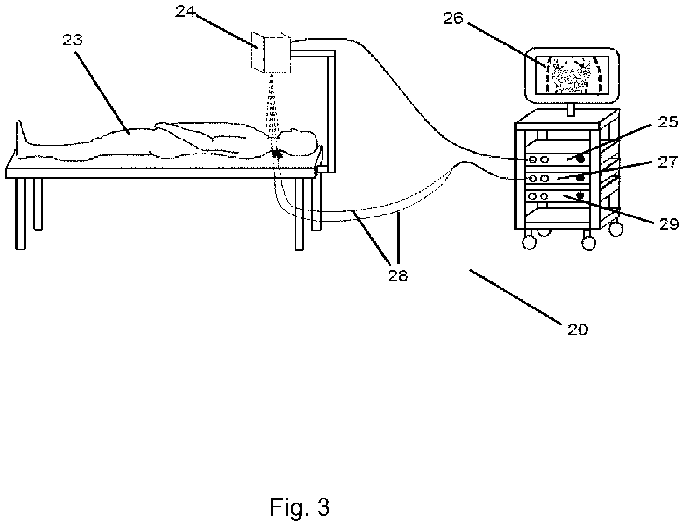

Fig. 3 zeigt schematisch ein erfindungsgemäßes Vorrichtungssystems wie im allgemeinen Teil beschrieben als Schema Vorrichtungssystem **20** während einer Operation an einem Patienten **23** - einzelne besondere Bestandteile des Vorrichtungssystems wie eine Einheit mit Scanner und Kamera **24,** eine Laserquelle **25** (als Beispiel eines Elementes einer Einheit zur Steuerung und/oder einer Quelle elektromagnetischer Strahlung), eine Anzeige (Situs mit Nervenverlauf) **26** als Beispiel eines Elementes einer Einheit zum Darstellen), die auch anders, insbesondere als Brille mit Microdisplay für einen Operateur, ausgeführt sein kann, einen Verstärker **27** (als Beispiel für ein Element einer Einheit zum Ableiten) für Signale auf den Ableitungen **28** (insbesondere von EMGs, ferner EEGs, EPs oder MEPs) und einen optionalen zusätzlichen elektrischen Stimulator **29** (als Beispiel für einen Bestandteil für die Ansteuerung einer Sonde für elektrische Impulse), der eine hier nicht gezeigte Handsonde mit Einzelelektrode oder eine Elektrodenarray ansteuern kann sind speziell dargestellt und fallen unter die allgemeine Darstellung (insbesondere in Absatz (A) oben), die hier im Beispiel ansonsten vorzugsweise uneingeschränkt gilt.

### Beispiel 4: Erfindungsgemäßes Vorrichtungssystem zur Ermittlung des Ortes von Nervengewebe-Arealen (insbesondere motorischen Arealen im Kortex) bei einer Operation am geöffneten Schädel

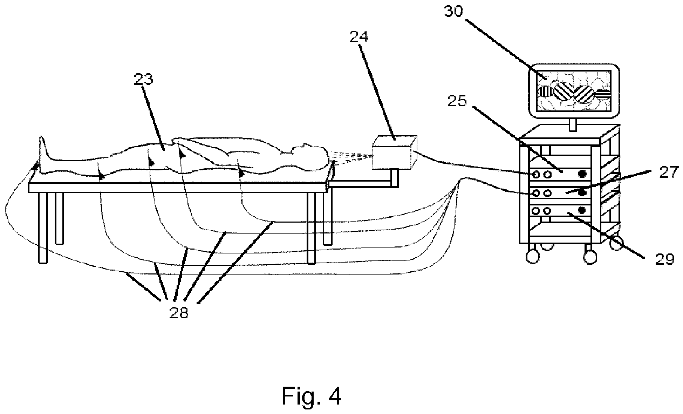

Fig. 4 zeigt schematisch ein erfindungsgemäßes Vorrichtungssystems wie im allgemeinen Teil beschrieben als Schema Vorrichtungssystem **20** während einer Operation am offenen Schädel eines Patienten **23** - einzelne besondere Bestandteile des Vorrichtungssystems wie eine Einheit mit Scanner und Kamera **24**, eine Laserquelle **25** (als Beispiel eines Elementes einer Einheit zur Steuerung und/oder einer Quelle elektromagnetischer Strahlung), eine Anzeige (Situs mit markierten funktionellen Arealen) **26** als Beispiel eines Elementes einer Einheit zum Darstellen), die auch anders, insbesondere als Brille mit Microdisplay für einen Operateur, ausgeführt sein kann, einen Verstärker **27** (als Beispiel für ein Element einer Einheit zum Ableiten) für Signale auf den Ableitungen **28** (insbesondere von EMGs, ferner EEGs, CEPs oder MEPs) und eine optionale zusätzliche Quelle für elektrische Stimulierung **29** (als Beispiel für einen Bestandteil für die Ansteuerung einer Sonde für elektrische Impulse), der eine hier nicht gezeigte Handsonde mit Einzelelektrode oder eine Elektrodenarray ansteuern kann sind speziell dargestellt und fallen unter die allgemeine Darstellung (insbesondere in Absatz (A) oben), die hier im Beispiel ansonsten vorzugsweise uneingeschränkt gilt.

### Beispiel 5: AR-Brille als erfindungsgemäßes Beispiel für eine Einheit zum Darstellen

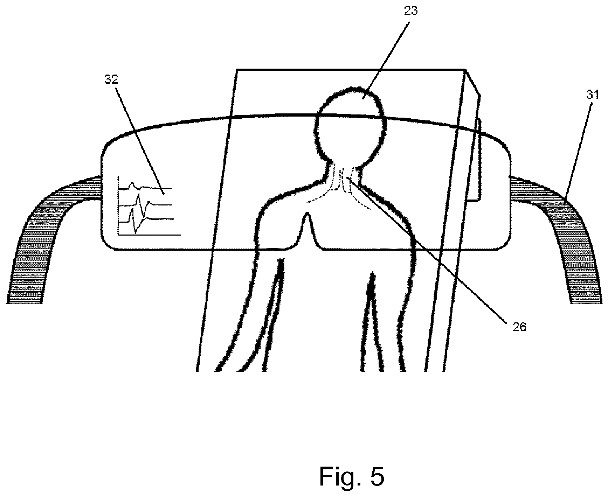

Als Beispiel für ein Element einer Einheit zum Darstellen (hier Visualisieren) der mit einem erfindungsgemäßen Vorrichtungssystem erhaltenen elektrophysiologischen Signale (EEG, EP, MEP oder insbesondere EMG) zeigt Fig. 5 eine Brille für erweiterte Wirklichkeit (AR-Brille) **31.** Vor dem Hintergrund des auf dem OP-Tisch gezeigten Patienten **23,** hier mit Operationssitus im Bereich von Schilddrüse, Nebenschilddrüse und Kehlkopf, blendet diese den Verlauf von Nerven (z.B. Nervus laryngeus recurrens, Teile des Nervus vagus oder des Nervus laryngeus superior) auf einem integrierten durchsichtigen Bildschirm, durch den der Operateur eine Anzeige **26** (Situs mit Nervenverlauf) direkt auf den OP-Situs eingeblendet bekommt. Er kann also direkt den OP-Situs und den dazu auf der AR-Brille eingeblendeten Nervenverlauf am richtigen Ort überlagert sehen. Beispielsweise seitlich ist im vorliegenden Beispiel auch noch der Verlauf von EMG-Signalen mittels einer EMG-Anzeige eingeblendet, die zusätzliche für die Operation relevante Informationen bereitstellt (z.B. über Form (z.B. Intensität) und Latenz/Dauer der dargestellten Signale). Derartige relevante Informationen können allgemeiner beispielsweise Informationen ausgewählt aus einer Amplitude oder zusätzlich Latenz des oder der elektrophysiologischen Signale, Parametern der Nervenreizauslösung (Stimulierung), der Tiefeninformation eines lokalisierten Nervs in Relation zur Oberfläche des Operationsbereiches, der Nähe einer Sonde in Relation zum Nerv, der Variation einer Amplitude oder zusätzlich Latenz des oder der elektrophysiologischen Signale über die Zeit, der Variation von Parametern der Nervenreizauslösung über die Zeit oder zwei oder mehr oder alle dieser Informationen sein, insbesondere unter Darstellung mittels eines Farbcodes, mittels numerischer Werte, mittels grafischer Elemente, mittels eingeblendeten Textes und/oder mittels Markierungszeichen neben oder auf dem Nervenverlauf oder dem Operationsbereich überlagert.

Eine derartige AR-Brille stellt auch für sich alleine eine besondere Ausführungsform der Erfindung dar, insbesondere jedoch als Bestandteil eines erfindungsgemäßen Vorrichtungssystems.

### Beispiel 6: Beispiel für einen optischen Aufbau und eine Ablenkeinheit für elektromagnetische (z.B. Laser-) Strahlung als Teil eines erfindungsgemäßen Vorrichtungssystems

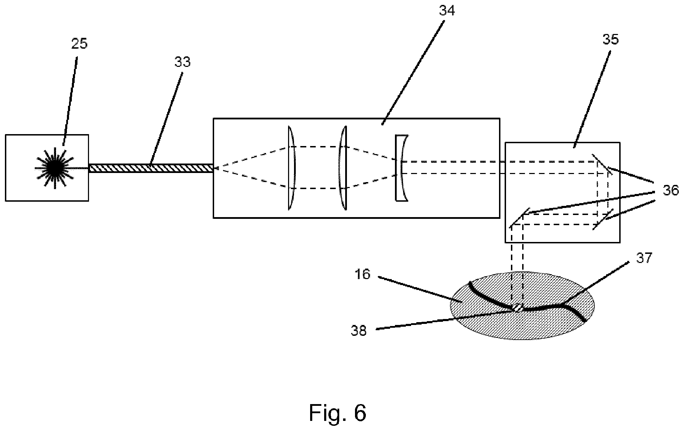

Fig. 6 zeigt eine schematische Darstellung eines exemplarischen optischen Aufbaus und einer exemplarischen Ablenkeinheit für elektromagnetische Strahlung, wie sie zur Kollimation und Fokussierung elektromagnetischer Strahlung und Steuerung des Einstrahlpunktes von Stimulationsreizen auf Basis elektromagnetischer Strahlung und/oder für die Verwendung entsprechender Strahlung aus dem sichtbaren Bereich zur Markierung (Überlagerung) des Verlaufes von Nerven oder des Ortes von Nervengeweben verwendet werden kann.

Diese beinhaltet eine Strahlungs-, insbesondere Laserquelle **25,** deren Strahlung über eine optische Faser **33** einem Linsenaufbau zur Kollimation **34** zugeleitet wird, von wo die gebündelte Strahlung einem Scanner **35** zugeführt wird, in dem ansteuerbare Spiegel **36** eine Positionierung des Einstrahlungspunktes (Stimulationsstelle **38**) unter Wirkung einer nicht gezeigten Einheit zur Steuerung einer Quelle nervenreizauslösender Strahlung im Situs **16** ermöglichen unter Darstellung eines mittels Abtastung mit einer derartigen Ablenkeinheit und Korrelation resultierender elektrophysiologischer Signale (wie z.B. EMG oder andere bereits genannte) erhaltenen Nervenverlaufes **37** (beispielsweise ebenfalls mittels (dann sichtbarer) Strahlung aus der Quelle **35** oder einer anderen Quelle von sichtbarem Licht) ermöglichen.

Die Erfindung betrifft in einer bevorzugten Ausführungsform ein erfindungsgemäßes Vorrichtungssystem, insbesondere wie oben als besondere Ausführungsform gekennzeichnet, in dem eine derartige Ablenkeinheit vorliegt.

### Beispiel 7: Erfindungsgemäße oder in einem erfindungsgemäßen Vorrichtungssystem vorliegende Handsonde für die Laserstimulation

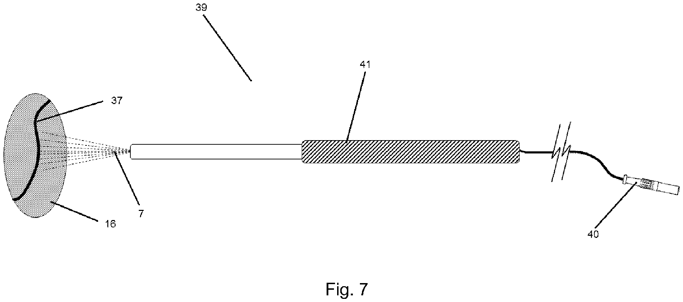

Fig. 7 zeigt eine als solche erfindungsgemäße oder in einem erfindungsgemäßen Vorrichtungssystem wie oben beschrieben als Element vorliegende Handsonde für die Laserstimulation. Diese beinhaltet ein Handstück **41,** einen Schaft, aus dem Laserlicht (Laser 7) austritt und auf einen Ort im Situs **16** gerichtet werden kann und einen Anschluss an eine Laserquelle **40,** wobei der Verlauf eines Nerven **37** über Korrelation aus der Reizung am Ort des Eintreffens von die Nerven stimulierenden Laserstrahlung über oben bereits erläuterte Einheiten und Elemente gewonnener elektrophysiologischer Signale ermöglicht werden kann.

Vorzugsweise kann die Handsonde zusätzlich mit einer Kameraeinheit überwacht werden, die eine genaue Lagebestimmung ermöglicht.

### Beispiel 8: Erfindungsgemäße oder in einem erfindungsgemäßen Vorrichtungssystem vorliegende Handsonde für elektrische Stimulation (oder Laserstimulation) mit Erfolgsanzeige:

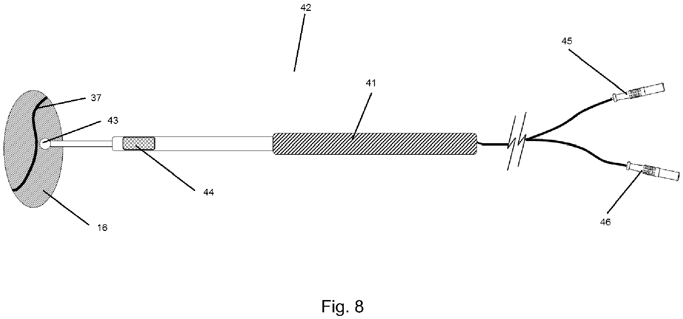

Fig. 8 zeigt eine als solche erfindungsgemäße oder in einem erfindungsgemäßen Vorrichtungssystem wie oben beschrieben als Element vorliegende Handsonde mit Erfolgsanzeige **42** für die elektrische Stimulation (alternativ kann sie auch zur Laserstimulation analog der in Beispiel 7 genannten Handsonde ausgerüstet sein). Diese beinhaltet ein Handstück **41**, einen Schaft, an dessen Ende ein elektrischer Stimulationskontakt **43** vorliegt, einen Anschluss **45** für die Stimulationselektrode, über die diese mit Stimulationsströmen versorgt wird, und zur Anzeige des Erfolgsfalles (positives elektrophysiologisches Signal, z.B. EMG, durch die Stimulation eines Nerven) eine LED zur Erfolgsanzeige **44** (oder ein anderes Leuchtelement, z.B. OLED, Lämpchen) am Schaft und einen Anschluss für die LED **46,** womit der Verlauf eines Nerven **37** im Situs **16** über Korrelation der Reizung am Ort des Eintreffens von die Nerven stimulierenden elektrischen Stimulationsreizen über oben bereits erläuterte Einheiten und Elemente gewonnener elektrophysiologischer Signale ermöglicht werden kann.

Mittels dieser Handsonde kann der Nervenverlauf oder der Ort eines Nervengewebes besonders einfach durch Abfahren und Aufleuchten der LED im Erfolgsfall nachvollzogen oder bei zusätzlicher AR-Darstellung bestätigt werden.

### Beispiel 9: Erfindungsgemäße oder in einem erfindungsgemäßen Vorrichtungssystem vorliegende Handsonde für elektrische Stimulation (oder Laserstimulation) mit alternativer Erfolgsanzeige:

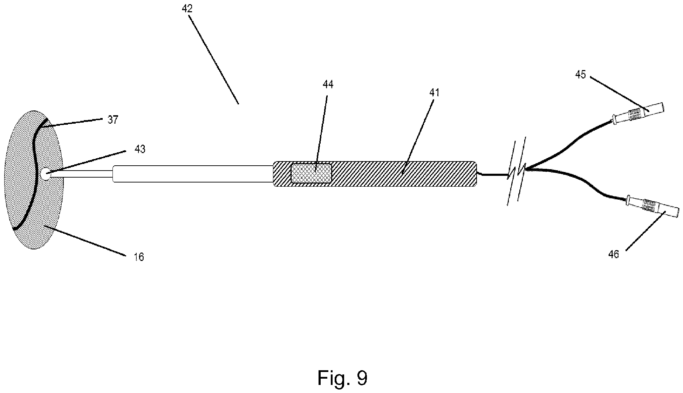

Fig. 9 zeigt eine als solche erfindungsgemäße oder in einem erfindungsgemäßen Vorrichtungssystem wie oben beschrieben als Element vorliegende Handsonde **42** mit zum in Fig. 7 gezeigten Falle alternativer Erfolgsanzeige für die elektrische Stimulation (alternativ kann sie auch zur Laserstimulation analog der in Beispiel 7 genannten Handsonde ausgerüstet sein). Diese beinhaltet ein Handstück **41,** einen Schaft, an dessen Ende ein elektrischer Stimulationskontakt **43** vorliegt, einen Anschluss **45** für die Stimulationselektrode, über die diese mit Stimulationsströmen versorgt wird, und zur Anzeige des Erfolgsfalles (positives elektrophysiologisches Signal, z.B. EMG, durch die Stimulation eines Nerven) diesmal am Handstück **41** eine LED zur Erfolgsanzeige **44** (oder ein anderes Leuchtelement, z.B. OLED, Lampe) mit einem Anschluss für die LED, womit der Verlauf eines Nerven **37** im Situs **16** über Korrelation der Reizung am Ort des Eintreffens von die Nerven stimulierenden elektrischen Stimulationsreizen über oben bereits erläuterte Einheiten und Elemente gewonnener elektrophysiologischer Signale ermöglicht werden kann.

Mittels dieser Handsonde kann der Nervenverlauf oder der Ort eines Nervengewebes besonders einfach durch Abfahren und Aufleuchten der LED im Erfolgsfall nachvollzogen oder bei zusätzlicher AR-Darstellung bestätigt werden.

### Beispiel 10: Erfindungsgemäße oder in einem erfindungsgemäßen Vorrichtungssystem vorliegende Handsonde für elektromagnetische oder Laserstimulation mit Licht zur Markierung eines Nervenverlaufs oder eine Nervengewebsortes (und/oder zur Beleuchtung des beaufschlagten Situsbereiches):

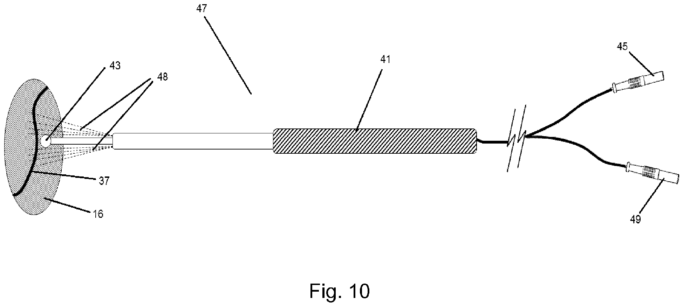

Fig. 10 zeigt eine als solche erfindungsgemäße oder in einem erfindungsgemäßen Vorrichtungssystem wie oben beschrieben als Element vorliegende Handsonde mit Anzeige des Nervenverlaufs **37** eines stimulierten Nerven oder von stimuliertem Nervengewebe (alternativ kann sie auch zur Laserstimulation analog der in Beispiel 7 genannten Handsonde ausgerüstet sein). Diese beinhaltet ein Handstück **41,** einen Schaft, an dessen Ende ein elektrischer Stimulationskontakt **43** vorliegt, einen Anschluss **45** für die Stimulationselektrode, über die diese mit Stimulationsströmen versorgt wird, und zur Anzeige des Erfolgsfalles (positives elektrophysiologisches Signal, z.B. EMG, durch die Stimulation eines Nerven) diesmal Licht zur Markierung **48** (welches über einen Anschluss an eine Lichtquelle **49** zugeführt werden kann), wobei der Verlauf eines Nerven **37** im Situs **16** durch das Licht zur Markierung **48** über Korrelation der Reizung am Ort des Eintreffens von die Nerven stimulierenden elektrischen Stimulationsreizen über oben bereits erläuterte Einheiten und Elemente gewonnener elektrophysiologischer Signale ermöglicht werden kann.

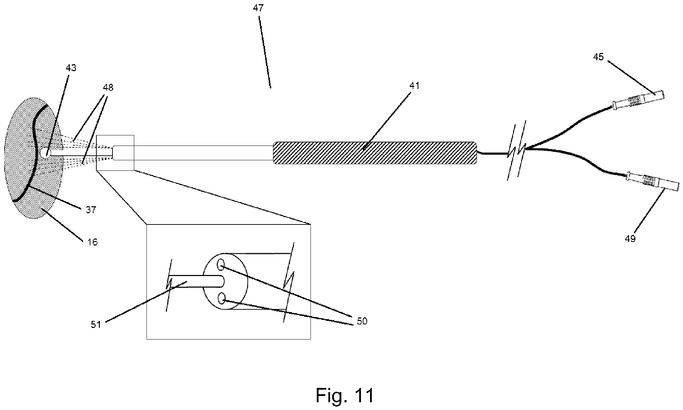

Fig. 11 zeigt exemplarisch einen Detailabschnitt mit einem Ausschnitt aus einem Teilstück **51** zum elektrischen Stimulationskontakt **43** und des Handstückes **41,** wo das Licht zur Markierung **48** aus Öffnungen für den Austritt des Lichtes **50** austreten kann.

Mittels dieser Handsonden kann der Nervenverlauf oder der Ort eines Nervengewebes besonders einfach durch Abfahren und Aufleuchten des Lichtes zur Markierung im Erfolgsfall der Stimulation nachvollzogen oder bei zusätzlicher AR-Darstellung bestätigt werden.

### Beispiel 11: Erfindungsgemäße oder in einem erfindungsgemäßen Vorrichtungssystem vorliegende Handsonde für die Laserstimulation mit Tracking-Marker

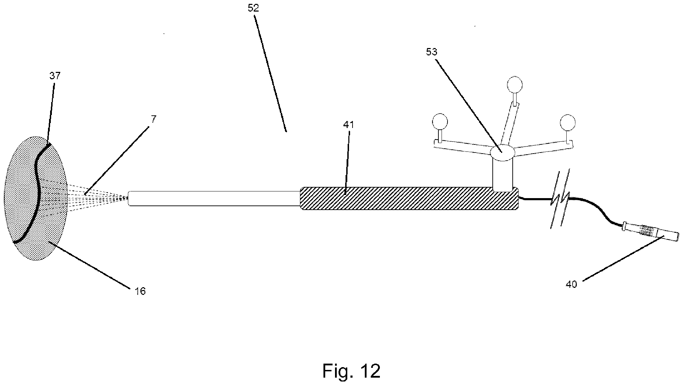

Fig. 12 zeigt eine als solche erfindungsgemäße oder in einem erfindungsgemäßen Vorrichtungssystem wie oben beschrieben als Element vorliegende Handsonde **52** für die Laserstimulation. Diese beinhaltet ein Handstück **41,** einen Schaft, aus dem Laserlicht **59** austritt und auf einen Ort im Situs **16** gerichtet werden kann und einen Anschluss an eine Laserquelle **40,** wobei der Verlauf eines Nerven **37** über Korrelation aus der Reizung am Ort des Eintreffens von die Nerven stimulierenden Laserstrahlung über oben bereits erläuterte Einheiten und Elemente gewonnener elektrophysiologischer Signale ermöglicht werden kann. Zusätzlich zur in Beispiel 7 gezeigten Handsonde (Fig. 7) findet sich (im Beispiel aber nicht zwingend an dieser Stelle) am Handstück **41** ein Marker für das optische Tracking **53,** beispielsweise für Tracking mittels IR und/oder Kameradarstellung, der jedoch alternativ auch für elektromagnetisches Tracking verwendet werden kann.

Mit Hilfe dieses Markers für das optische Tracking **53** kann die Handsonde **52** derart überwacht werden, dass eine genaue Lagebestimmung ermöglicht wird und als Basis für die Ermittlung und Darstellung des Nervenverlaufes oder des Ortes eines Nervengewebes mittels eines erfindungsgemäßen, wie oben beschriebenen Vorrichtungssystems dient.

### Beispiel 12: Darstellung eines Algorithmus zur beschleunigten Bestimmung des Verlaufes eines Nerven oder eines Nervengewebsortes innerhalb eines erfindungsgemäßen Vorrichtungssystems

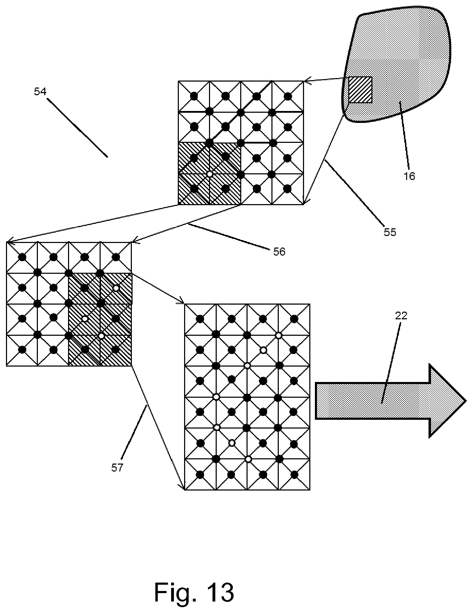

Fig. 13 zeigt in schematischer Darstellung einen Algorithmus, wie er in einem erfindungsgemäßen Vorrichtungssystem implementiert sein kann.

Beispielsweise mit einem Scanner oder einer navigierbaren Stimulationssonde wie oben dargestellt können mittels eines groben Abtastens mit einem Stimulationsreiz in einem Schritt I **55** Areale determiniert werden, in denen überhaupt eine Reizung (durch rückkehrendes elektrophysiologisches Signal) gefunden wird (dargestellt durch graue Rasterung). In einem Schritt II **56** wir das so eingegrenzte Areal erneut in feinerer Auflösung abgetastet, was eine weitere Präzisierung des Areals (ebenfalls grau gerastert), in dem Reizung gefunden wird, ermöglicht. Ein Schritt III **57** ermöglicht ein noch feineres Abtastmuster, mit dem schließlich beispielsweise ein Nervenverlauf genau bestimmt und für die Darstellung in der Vorrichtungseinheit bereitgestellt werden kann. Ausgefüllte Kreise bedeuten in der Zeichnung Abtastpunkte des Lasers (Einstrahlungspositionen), offene Kreise Stellen, an denen die Einstrahlung zu detektierter elektrophysiologischer Antwort (z.B. EMG-Antwort) geführt hat, also Orte erfolgreicher Stimulation.

Es können auch mehr oder weniger Schritte verwendet werden, und analog können auch Nervengewebe iterativ eingegrenzt werden. Die Areale können auch andere Formen als dargestellt aufweisen - die Abbildung dient nur der Veranschaulichung des Prinzips des Algorithmus.

**Liste der Bezugszeichen**

| Nummer des Bezugszeichens | Bedeutung |
|---|---|
| 1 | Vorrichtungssystem (erfindungsgemäß) |
| 2 | Kontrolleinheit mit Einrichtung zur Darstellung von Nervenbahnen |
| 3 | Bilddatenübertragung |
| 4 | Laserparameterübertragung |
| 5 | Positionsdatenübertragung |
| 6 | Kamera |
| 7 | Laser |
| 8 | Lasermarkierer |
| 9 | Scanner |
| 10 | Funktionell relevantes Areal |
| 11 | Zeitinformationsübertragung |
| 12 | Zielorgan |
| 13 | Verstärker |
| 14 | Erfolgsinformationsübertragung |
| 15 | Stimulus |
| 16 | Situs |
| 17 | Zähler |
| 18 | EMG-Ableitung |
| 19 | Optische Erfassung |
| 20 | Schema Vorrichtungssystem |
| 21 | IONM-System |
| 22 | Darstellung |
| 23 | Patient |
| 24 | Scanner und Kamera |
| 25 | Laserquelle |
| 26 | Anzeige (Situs mit Nervenverlauf) |
| 27 | Verstärker |
| 28 | Ableitung |
| 29 | Quelle für elektrische Stimulierung |
| 30 | Anzeige (Kortex mit markierten Arealen) |
| 31 | AR-Brille |
| 32 | Anzeige von EMG-Signalen |
| 33 | optische Faser |
| 34 | Linsenaufbau zur Kollimation |
| 35 | Scanner |
| 36 | Spiegel |
| 37 | Nerv |
| 38 | Stimulationsstelle |
| 39 | Handsonde für Laserstimulation |
| 40 | Anschluss an die Laserquelle |
| 41 | Handstück |
| 42 | Stimulationssonde mit LED zur Erfolgsanzeige |
| 43 | elektrischer Stimulationskontakt |
| 44 | LED zur Erfolgsanzeige |
| 45 | Anschluss Stimulationselektrode |
| 46 | Anschluss LED |
| 47 | Stimulationssonde zur Markierung mit Licht |
| 48 | Licht zur Markierung |
| 49 | Anschluss Lichtquelle |
| 50 | Öffnungen für den Austritt des Lichts |
| 51 | Teilstück zu Stimulationskontakt |
| 52 | Getrackte Handsonde für die Laserstimulation |
| 53 | Marker für das optische Tracking |
| 54 | Abtastalgorithmus |
| 55 | Schritt I |
| 56 | Schritt II |
| 57 | Schritt III |

## Patentansprüche

1. Vorrichtungssystem zur intraoperativen Ortung von Nervenbahnen, welches zur automatisierten intraoperativen Messung der Reizbarkeit von Nerven oder Nervengewebe und zur Darstellung des Verlaufes eines oder mehrerer Nerven oder der Lage eines Nervengewebes eingerichtet ist und einen oder mehrere Sensoren zur Ableitung von ein oder mehreren elektrophysiologischen Signalen, die als Resultat einer Reizung von Nerven stimulierter Organe auftreten, umfasst, wobei dieses System
- eine Einheit zur Steuerung einer Quelle von nervenreizauslösender elektromagnetischer Strahlung mit einer Wellenlänge im Bereich von 500 nm bis 3000 nm zur Reizung von Nerven und/oder einer Quelle elektrischer Impulse zur Nervenreizauslösung mittels elektrischer Impulse;
- eine Einheit zum Ableiten durch die Nervenreizauslösung hervorgerufener des oder der elektrophysiologischer Signale,
- eine Einheit zum Berechnen, Steuern und Auswerten und
- eine Einheit zum Darstellen, insbesondere Visualisieren und/oder zum akustischen Darstellen des oder der elektrophysiologischen Signale beinhaltet,
wobei zwei oder mehr der genannten Einheiten auch zu einer übergeordneten Einheit zusammengefasst sein können, und
**dadurch gekennzeichnet ist, dass** es
- eine Quelle der elektromagnetischen Strahlung (für elektromagnetische (Stimulations-) Impulse) umfasst, die eine oder mehr linear oder zwei- oder dreidimensional verfahrbare Einzelquellen und/oder ein Array mehrerer Quellen elektromagnetischer Strahlung beinhaltet oder eine lokalisierbare Sonde als Quelle elektromagnetischer Strahlung beinhaltet; und vorzugsweise eine Ablenkeinheit für die elektromagnetische Strahlung der ein oder mehreren Quellen elektromagnetischer Strahlung vorliegt, um die elektromagnetische Strahlung gezielt in intraoperativ zu untersuchende Bereiche einzustrahlen,
- und/oder eine lokalisierbare Sonde als die Quelle elektrischer (Stimulations-) Impulse beinhaltet,
und dass es derart eingerichtet ist, dass die Visualisierung und/oder akustische Darstellung des oder der elektrophysiologischen Signale und so des Verlaufs mindestens eines Nerven und/oder des Ortes eines Nervengewebes im Operationsbereich (in situ) oder zusätzlich oder alleine unter Darstellung von zusätzlichen für die Operation relevanten weiteren Informationen erfolgt, und dass die Visualisierung mittels einer Vorrichtung für erweiterte oder gemischte Realität erfolgt.

2. Vorrichtungssystem nach Anspruch 1, **dadurch gekennzeichnet, dass** es zur Visualisierung unter Darstellung von zusätzlichen relevanten Informationen ausgewählt aus einer Amplitude oder zusätzlich Latenz des oder der elektrophysiologischen Signale, Parametern der Nervenreizauslösung, der Tiefeninformation eines lokalisierten Nervs in Relation zur Oberfläche des Operationsbereiches, der Nähe einer Sonde in Relation zum Nerv, der Variation einer Amplitude oder zusätzlich Latenz des oder elektrophysiologischen Signale über die Zeit, der Variation von Parametern der Nervenreizauslösung über die Zeit oder zwei oder mehr oder aller dieser Informationen eingerichtet ist, insbesondere unter Darstellung mittels eines Farbcodes, mittels numerischer Werte, mittels grafischer Elemente, mittels eingeblendeten Textes und/oder mittels Markierungszeichen neben oder auf dem Nervenverlauf oder auf dem Operationsbereich.

3. Vorrichtungssystem nach einem der vorstehenden Ansprüche, **dadurch gekennzeichnet, dass** es eine Vorrichtung für erweiterte oder gemischte Realität, ausgewählt aus einer Brille mit einem Microdisplay, einem 3D-Projektor und einem 2D Projektor, wobei die Brille mit einem integrierten Laserschutz ausgestattet sein kann, oder ferner eine Anbindung an einen Laparoskopie-Bildschirm, einen Bildschirm eines Operationsroboters oder ein Operations-Mikroskop enthält, und die Darstellung von für die Operation relevanten Informationen durch Einblendung der zusätzlichen Informationen auf ein (z.B. Video-) Bild des ermittelten Nervenverlaufes erfolgt.

4. Vorrichtungssystem nach einem der vorstehenden Ansprüche, **dadurch gekennzeichnet, dass** als Sensor(en) zur Ableitung des oder der elektrophysiologischen Signale ein oder mehrere solche für mindestens ein elektrophysiologisches Signal, insbesondere Elektromyogramm (EMG), umfasst sind und es somit für die Ableitung von ein oder mehreren elektrophysiologischen Signalen von glatter und/oder quergestreifter Muskulatur nach Reizung von peripheren oder zentralen Nerven eingerichtet ist; oder
**dadurch gekennzeichnet, dass** als Sensor(en) zur Ableitung der physiologischen Signale ein oder mehrere solche für mindestens ein elektrophysiologisches Signal, insbesondere ein Evoziertes Potential (EP), umfasst sind und es somit für die Ableitung von ein oder mehreren elektrophysiologischen Signalen nach Reizung von peripherem oder zentralem Nervengewebe eingerichtet ist.

5. Vorrichtungssystem nach einem der vorstehenden Ansprüche, (a) **dadurch gekennzeichnet, dass** es als lokalisierbare Sonde zum Abgeben elektrischer (Stimulations-) Impulse eine lokalisierbare Handsonde mit mindestens einer Elektrode und/oder eine die Bestimmung des Ortes einer elektrischen Stimulation ermöglichende Elektrodenmatrix beinhaltet; oder
(b) **dadurch gekennzeichnet, dass** es als lokalisierbare Sonde zum Abgeben von elektromagnetischer Strahlung eine lokalisierbare Handsonde mit mindestens einer Quelle und/oder eine die Bestimmung des Ortes einer Stimulation durch elektromagnetische Strahlung ermöglichende Elektrodenmatrix beinhaltet.

6. Vorrichtungssystem nach einem der vorstehenden Ansprüche, **dadurch gekennzeichnet, dass** es als Quelle der elektromagnetischen Strahlung einen Laser umfasst, insbesondere einen Feststofflaser, z.B. Nd/YAG-Laser, Titan:Saphir-Laser, Faserlaser und/oder insbesondere mindestens eine Laserdiode, oder worin
der oder die Laser Gaslaser, z.B. Helium-Neon-Laser, Kohlendioxidlaser, Metalldampflaser oder Farbstofflaser, sind; oder worin der oder die Laser Freie-Elektronen-Laser sind.

7. Vorrichtungssystem nach Anspruch 6, **dadurch gekennzeichnet, dass** es sich bei der Ablenkeinheit der elektromagnetischen Strahlung um akustooptische Deflektoren, elektrooptische Deflektoren, Flash-LiDAR, einen Prismenscanner oder insbesondere einen Spiegelscanner mit Galvanometerantrieb handelt.

8. Vorrichtungssystem nach Anspruch 7, **dadurch gekennzeichnet, dass** die Ablenkeinheit eingerichtet ist zur automatischen Abrasterung des interessierenden Bereichs durch die elektromagnetische Strahlung.

9. Vorrichtungssystem nach Anspruch 8, **dadurch gekennzeichnet, dass** der Nervenverlauf durch den interessierenden Bereich durch Korrelation der Ortsinformation der Ablenkeinheit mit dem mittels des oder den Sensoren abgeleiteten Signal berechnet wird.

10. Vorrichtungssystem nach einem der vorstehenden Ansprüche **dadurch gekennzeichnet, dass** die Vorrichtung zur Visualisierung des Nervenverlaufs im Operationsbereich eine Lichtquelle, insbesondere in Form eines Laser-Projektors oder eines Pilotlasers, umfasst.

11. Vorrichtungssystem nach einem der vorstehenden Ansprüche, **dadurch gekennzeichnet, dass** es ein oder mehrere Linsen zur Kollimation der elektromagnetischen Strahlen, insbesondere von Laserlicht, umfasst.

12. Vorrichtungssystem nach Anspruch 11, **dadurch gekennzeichnet, dass** es ein oder mehrere Linsen zur Formung eines Gauß-Stahls und eine Einrichtung zur Verschiebung der einen oder mehreren Linsen zur Verschiebung des Fokuspunktes der elektromagnetischen Strahlen, insbesondere von Laserlicht, in verschiedenen Tiefen des Operationsbereichs umfasst und so eine 3D-Darstellung der zu reizenden Nerven ermöglicht.

13. Vorrichtungssystem nach Anspruch 11, **dadurch gekennzeichnet, dass** es ein oder mehrere Linsen zur Formung eines Bessel-Gauß-Strahls der elektromagnetischen Strahlen, insbesondere von Laserlicht umfasst.

14. Vorrichtungssystem nach Anspruch 12 oder 13, **dadurch gekennzeichnet, dass** es eine Einrichtung zur Änderung der Leistung der elektromagnetischen Strahlung umfasst und so eine Ausbreitung des Stimulationseffektes in verschiedene Tiefen und eine 3D-Darstellung der zu reizenden Nerven ermöglicht.

15. Vorrichtungssystem nach einem der Ansprüche 1 bis 5, **dadurch gekennzeichnet, dass** die lokalisierbare Handsonde ein oder mehrere Anzeigeelemente aufweist, welche die Darstellung der relevanten weiteren Informationen ermöglichen, z.B. auf einem Bildschirm oder mittels ein oder mehrerer LEDs; oder dass sie mindestens eine Lichtquelle, insbesondere in Form von ein oder mehreren LEDs oder einem Pilotlaser, umfasst zur Ausgabe von Signalen mittels sichtbarem Licht, basierend auf den abgeleiteten physiologischen Signalen, die als Resultat einer Reizung von Nerven auftreten.
